Europäisches Patentamt

European Patent Office

Office européen des brevets

Veröffentlichungsnummer: **0 286 781**
**A2**

# EUROPÄISCHE PATENTANMELDUNG

Anmeldenummer: 88100970.8

Anmeldetag: 22.01.88

Int. Cl.4 **A61K 45/00 , A61K 33/10 ,**
**A61K 33/12 , A61K 33/06 ,**
**//(A61K33/10,33:08,31:415),**
**(A61K33/12,33:10,33:08,**
**31:415),(A61K33/06,31:415),**
**(A61K33/10,33:08,31:345),**
**(A61K33/10,33:08,31:345)**

Priorität: 30.03.87 DE 3710462

Veröffentlichungstag der Anmeldung:
**19.10.88 Patentblatt 88/42**

Benannte Vertragsstaaten:
**AT BE CH DE FR GB IT LI LU NL SE**

Anmelder: **HEUMANN PHARMA GMBH & CO**
**18-28 Heideloffstrasse**
**D-8500 Nürnberg(DE)**

Erfinder: **Schickaneder, Helmut, Dr.**
**Dipl.-Chem.**
**Moosäcker 25**
**D-8501 Eckental(DE)**
Erfinder: **Vergin, Hartmut, Dr. Dipl.-Biochem.**
**Ebenreuther Strasse 32**
**D-8500 Nürnberg 30(DE)**
Erfinder: **Grätzel von Grätz, Jochen, Dr.**
**Dipl.-Chem.**
**Ulmenstrasse 29**
**D-8501 Feucht(DE)**
Erfinder: **Ahrens, Kurt Henning, Dr.**
**Praterstrasse 9**
**D-8500 Nürnberg(DE)**

Vertreter: **Kraus, Walter, Dr. et al**
**Patentanwälte Kraus, Weisert & Partner**
**Thomas-Wimmer-Ring 15**
**D-8000 München 22(DE)**

**Pharmazeutische Zubereitung zur Behandlung von Erkrankungen des Magen-Darm-Traktes.**

Beschrieben wird eine pharmazeutische Zubereitung mit cytoprotektiver Wirkung auf den Gastrointestinaltrakt, die eine Kombination aus einem Histamin-$H_2$-Rezeptorantagonisten und einem antaziden Stoff sowie einen pharmazeutisch annehmbaren Träger enthält.

EP 0 286 781 A2

Die vorliegende Erfindung betrifft eine pharmazeutische Zubereitung, die eine Kombination von Säuresekretionshemmern vom Typ der Histamin-$H_2$-Rezeptorantagonisten mit antaziden Stoffen, welche zu funktioneller Cytoprotektion bei Magen-Darm-Erkrankungen befähigt sind, darstellt.

Bestimmte Verbindungen, d.h. sogenannte Histamin-$H_2$-Rezeptorantagonisten, zeigen aufgrund ihrer spezifischen $H_2$-antagonistischen Aktivität eine Hemmung der Magensäuresekretion, wenn diese durch Histaminantagonisten stimuliert wird (Ash und Schild, "Brit. J. Pharmacol. Chemother.", 27, 427 (1966) und Black u.a., "Nature", 236, 385 (1971)). Die pharmakologische Aktivität dieser Verbindungen kann beispielsweise beim perfundierten Rattenmagen oder durch die Ermittlung der $pA_2$-Werte in vitro am Meerschweinchenvorhof (vgl. Ariens, "Molecular Pharmacology", Band 1, Academic Press, New York (1964)) nachgewiesen werden. Weiterhin kann die $H_2$-antagonistische Wirkung an wachen Heidenhain-Pouch-Hunden nach der Methode von Black u.a., "Nature, 236, 385 (1971) und an wachen Fistelkatzen gezeigt werden. Diese Verbindungen antagonisieren weiterhin die Histaminwirkung auf die Kontraktionsfrequenz des isolierten rechten Atriums des Meerschweinchens, aber sie beeinflussen nicht histamininduzierte Kontraktionen des isolierten, glatten gastrointestinalen Muskels, wenn diese durch $H_2$-Agonisten hervorgerufen werden.

Als wichtigste Vertreter der Histamin-$H_2$-Rezeptorantagonisten können Ranitidin (DE-OS 27 34 070) und Cimetidin (DE-OS 27 42 531) genannt werden.

Antacida sind pharmazeutische basische Stoffe, die bei oraler Anwendung zur ausreichenden Neutralisation der Magensäure befähigt sind, wie zum Beispiel Magnesiumoxid, -hydroxid, -carbonat, Aluminiumhydroxycarbonate, Aluminiumnatriumcarbonat, Aluminiummagensiumsilicathydrat, Aluminiumsucrosesulfat, Hydrotalcid, Magaldrat und Calciumcarbonat. Antacida dieser Art werden bereits in "Arzneimittel-Verf.", Ehrhart'Ruschig, Seite 757 (1968) beschrieben.

Überraschenderweise wurde nun gefunden, daß durch Kombination von Histamin-$H_2$-Antagonisten mit antaziden Stoffen der oben erwähnten Art eine überraschende Verstärkung der antiulcerogenen Wirkung des $H_2$-Antagonisten bewirkt wird, wodurch eine Verringerung seiner Dosis ermöglicht wird. Hierdurch werden therapeutische Vorteile, insbesondere eine Verminderung der Nebenwirkungn, erreicht.

Für die erfindungsgemäße pharmazeutische Zubereitung kommen insbesondere Ranitidin und Cimetidin in Betracht. Es können aber auch alle anderen therapeutisch einsetzbaren $H_2$-Rezeptorantagonisten, wie sie zum Beispiel in der DE-OS 34 41 086 beschrieben werden, verwendet werden.

Als Antacida kommen die oben beschriebenen Stoffe in Betracht. Bevorzugt werden antazide Substanzen, die zu einer funktionellen Cytoprotektion befähigt sind. Unter "funktioneller Cytoprotektion" versteht man u.a. die Wirkung bestimmer Antacida, die zu einem erhöhten $PGE_2$-Spiegel in Magenmukosa und Lumen führt und damit zu einem Schutz der Magenwand gegenüber exogenen und endogenen Noxen.

Als Antacidum mit dieser Wirkung kommt insbesondere Aluminiumhydroxidgel in Betracht. Diese Substanz kann auch in Kombination mit Calciumcarbonat bzw. mit Calciumcarbonat und Magnesiumhydroxid angewendet werden. Ein weiterer antazider Stoff, der zu eienr funktionellen Cytoprotektion im Sinne der Erfindung befähigt ist, ist auch ein basisches Aluminiumsucrosesulfat, das auch als "Sucralfat" bezeichnet wird. Die säureneutralisierende Wirkung von Sucralfat ist vergleichsweise gering.

Erfindungsgemäß wird eine Kombination aus Cimetidin oder Ranitidin und einer Mischung aus Aluminiumhydroxid, Magnesiumhydroxid und Calciumcarbonat bevorzugt. Letztere Mischung ist unter dem Warenzeichen "Trigastril" im Handel.

Bevorzugt werden weiterhin Kombinationen, die einerseits Cimetidin oder Ranitidin und andererseits eine Mischung aus folgenden Komponenten: Ma gnesiumoxid, Magnesiumhydroxid, Aluminiumhydroxycarbonat, Aluminiumnatriumcarbonat und/oder Aluminiummagnesiumsilicathydrat enthalten.

Bezogen auf die übliche Dosierung der Antacida von 20 bis 100 mVal Neutralisationskapazität bzw. 1 g Sucralfat, können 40 bis 60% der therapeutisch wirksamen Dosierung des $H_2$-Blockers als Monosubstanz eingesetzt werden:

Cimetidin 100 bis 500 mg

Ranitidin 100 bis 200 mg

Bevorzugte Kombinationen bestehen aus Antacida mit einer Neutralisationskapazität von 30 bis 80 mVal pro Dosis, wobei jeweils mehr als 50% der Neutralisationskapazität von Aluminiumhydroxidgel getragen werden, und $H_2$-Antagonist in der Dosierung von 100 bis 400 mg.

Die erfindungsgemäße pharmazeutische Zubereitung kann in an sich bekannter Weise durch Mischen der Einzelkomponenten in Form von peroralen Darreichungsformen, wie Tabletten, Lutschtabletten, Kapseln, Granulaten, Gelen und Suspensionen, hergestellt werden. Hierzu werden pharmazeutisch annehmbare Träger, wie Wasser, Zuckeralkohole, wie Sorbit und Mannit, pflanzliche oder halbsynthetische Triglyceride, wie Lactose, Rohrzucker und Traubenzucker, Stabilisatoren, wie Celluloseether, hochdisperse Kieselsäure, Gleitmittel, wie Magnesiumstearat und Talkum, Glykole, wie PEG, geschmacksbeeinflussende Stoffe, wie Süßstoffe und Aromen, sowie Konservierungsstoffe, wie zum Beispiel p-Hydroxybenzoesäureester, verwendet.

In den erfindungsgemäßen pharmazeutischen Zubereitungen liegt die Kombination aus Histamin-H$_2$-Rezeptorantagonist und antazidem Stoff in festen Darreichungsformen vorzugsweise in einer Konzentration von 10 bis 90 Gew.-%, in flüssigen oralen Darreichungsformen von 10 bis 80 Gew.-%, vor, um den angegebenen Dosierungsspielraum zu erreichen.

Eine Dosiseinheit der erfindungsgemäßen pharmazeutischen Zubereitung enthält 0,5 bis 1,5 g, vorzugsweise 0,8 bis 1,2 g, der Kombination aus Histamin-H$_2$-Rezeptorantagonist und antaziden Stoff. Die Dosiseinheit wird vorzugsweise ein-bis viermal täglich verabreicht.

Im Einzelfall kann es gegebenenfalls erforderlich sein, von den genannten Mengen abzuweichen, und zwar in Abhängigkeit vom individuellen Verhalten gegenüber der erfindungsgemäß verwendeten Kombination, dem Zustand des Patienten und ähnlicher Faktoren. So gibt es zum Beispiel Fälle, wo mit weniger als der oben genannten Mindestmenge ausgekommen werden kann, während in anderen Fällen die genannte obere Grenze überschritten werden muß.

Die Applikation erfolgt in üblicher Weise oral durch je nach Darreichungsform Lutschen, Kauen und Schlucken, gegebenenfalls nach Anrühren mit Wasser. Flüssige Zubereitungen können auch über eine Schlundsonde direkt in den Magen gegeben werden.

Die antiulcerogene Wirksamkeit der erfindungsgemäßen Kombination soll im folgendenden Beispiel der Kombination von Cimetidin und dem handelsüblichen Antacidum Trigastril® aufgezeigt werden.

Untersuchungen am Modell der immobilisationsstreßinduzierten gastromukosalen Schädigung der Ratte

Männlichen Sprague-Dawley-Ratten (SD-SIV, Fa. Savo, Kisslegg) der Gewichtsklasse 180 bis 200 g wurde für 16 Stunden das Futter (Altromin®, Standarddiät für Ratten/Mäuse (Fa. Altromin, Lage/Lippe) entzogen, während Leitungswasser ad libitum zur Verfügung stand. Kontrollratten wurden mit 7,5 ml/kg 1%iger Tylose (MH-300, Fa. Hoechst, Frankfurt) und Testtiere mit Trigastril, Cimetidin und der Kombination beider Substanzen - jeweils in 1%iger Tylose suspendiert - intragastral behandelt. Die Applikationsvolumina waren in allen Fällen gleich. Von hochdosiertem Cimetidin (ca. 50 mg/kg) ist die ulcus-bzw. cytoprotektive Wirkung 1984 beschrieben worden (A. Robert u.a. (1984), "Scand. J. Gastroenterol.", 19, (Suppl. 101), 69).

Gestreßt wurden die Tiere unmittelbar nach Applikation. Nach Versuchsende wurden den Tieren die Mägen in Ethernarkose entnommen. Die Mägen wurden entlang der großen Kurvatur eröffnet und gespült. Ausmaß und Beschaffenheit der Läsionen wurden nach einer von Marazzi-Uberti und Turba (C. Med. exp., 4, 284 (1961)) angegebenen Einteilung visuell ausgezählt und als Ulcus-Index ausgedrückt. Die Ergebnisse dieses Versuchs sind in der Tabelle zusammengestellt, die die protektive Wirkung von Cimetidin, Trigastril® sowie deren Kombination am Modell der streßinduzierten gastromukosalen Schädigung der Ratte zeigt.

| Substanz | Dosis mg/kg (ml/kg) | Tier-zahl | Ulcus-Index (MW $\pm$) SD | % Hemmung |
|---|---|---|---|---|
| 1% Tylose | - | 10 | 69,8 $\pm$ 10,7 | - |
| Cimetidin | 50 | 10 | 32,1 $\pm$ 6,6*) | 54 |
| Trigastril® | 4[2]) | 10 | 43,8 $\pm$ 7,6*) | 27 |
| Cimetidin + Trigastril® | 50 4[2]) | 10 | 12,2 $\pm$ 5,3*),°) | 82 |

*) signifikanter Unterschied zu tylosebehandelten Kontrolltieren (p 0,05)

°) signifikanter Unterschied Kombination gegenüber den Einzelsubstanzen (p 0,05)

[2]) 4 ml/kg Trigastril® entspricht ca. 0,8 ml Originalgel-Suspension pro Tier oder 620 mg $Al(OH)_3$-Gel (12,5%ig) pro Einzeldosis

Die Erfindung wird in den Beispielen erläutert.

**Beispiel 1**

<u>Herstellung einer Emulsion</u>

Es werden 10 ml einer Suspension mit folgender Zusammensetzung hergestellt:

| | | |
|---|---|---|
| Aluminiumhydroxidgel (10%, bezogen auf $Al_2O_3$) | 6,3 | g |
| Magensiumhydroxidpaste (30%, bezogen auf $Mg(OH)_2$) | 1,5 | g |
| Cimetidin | 0,4 | g |
| Sorbit | 1,5 | g |
| Glycerin | 0,2 | g |
| Methylparahydroxybenzoat | 0,01 | g |
| Propylparahydroxybenzoat | 0,005 | g |
| Pfefferminzaroma | 0,1 | g |
| Gereinigtes Wasser | 1,185 | g |
| | 11,200 | g |

Die Substanzen werden in üblicher Weise gemischt, und die Mischung wird homogenisiert. Sodann wird die Mischung in Einzeldosisbeutel verpackt. Dosierung: täglich 1 Beutel vor dem Schlafengehen

**Beispiel 2**

Herstellung von Tabletten

Es werden Tabletten mit folgender Zusammensetzung hergestellt:

| | |
|---|---|
| Aluminiumhydroxid-Trockengel (50%, bezogen auf $Al_2O_3$) | 400,0 mg |
| Calciumcarbonat | 600,0 mg |
| Ranitidin | 100,0 mg |
| Mannit | 479,0 mg |
| Karamelaroma | 5,0 mg |
| Magnesiumstearat | 16,0 mg |
| | 1600,0 mg |

Die Substanzen werden trocken gemischt und auf einer Rundläufer-Tablettiermaschine zu Tabletten mit 19 mm Durchmesser verpreßt. Dosierung:

**Ansprüche**

1. Pharmazeutische Zubereitung mit cytoprotektiver Wirkung auf den Gastrointestinaltrakt, dadurch **gekennzeichnet,** daß sie eine Kombination aus einem Histamin-$H_2$-Rezeptorantagonisten und einem antaziden Stoff, der zu einer funktionellen Cytoprotektion befähigt ist, und einen pharmazeutisch annehmbaren Träger enthält.

2. Pharmazeutische Zubereitung nach Anspruch 1, dadurch **gekennzeichnet,** daß sie als $H_2$-Rezeptorantagonist Cimetidin und als antaziden Stoff eine Mischung aus Aluminiumhydroxid, Magnesiumhydroxid und Calciumcarbonat enthält.

3. Pharmazeutische Zubereitung nach Anspruch 1, dadurch **gekennzeichnet,** daß sie als $H_2$-Rezeptorantagonist Cimetidin und als antaziden Stoff eine Mischung aus Magnesiumoxid, Magnesiumhydroxid, Aluminiumhydroxycarbonat, Aluminiumnatriumcarbonat und/oder Aluminiummagnesiumsilicathydrat enthält.

4. Pharmazeutische Zubereitung nach Anspruch 1, dadurch **gekennzeichnet,** daß sie als $H_2$-Rezeptorantagonist Cimetidin und als antaziden Stoff basisches Aluminiumsucrosesulfat enthält.

5. Pharmazeutische Zubereitung nach Anspruch 1, dadurch **gekennzxeichnet,** daß sie als $H_2$-Rezeptorantagonist Ranitidin und als Antacidum eine Mischung aus Aluminiumhydroxid, Magnesiumhydroxid und Calciumcarbonat enthält.

6. Pharmazeutische Zubereitung nach Anspruch 1, dadurch **gekennzeichnet,** daß sie als $H_2$-Rezeptorantagonist Ranitidin und als antaziden Stoff eine Mischung aus Magnesiumoxid, Magnesiumhydroxid, Aluminiumhydroxycarbonat, Aluminiumnatriumcarbonat und/oder Aluminummmagnesiumsilicathydrat enthält.

7. Pharmazeutische Zubereitung nach Anspruch 1, dadurch **gekennzeichnet,** daß sie als $H_2$-Rezeptorantagonist Ranitidin und als antaziden Stoff basisches Aluminiumsucrosesulfat enthält.

8. Verwendung einer Kombination aus einem Histamin-$H_2$-Rezeptorantagonisten und einem antaziden Stoff zur Herstellung eines Arzneimittels zur Behandlung von gastrointestinalen Erkrankungen.